# EUROPEAN PATENT APPLICATION

(11) **EP 1 709 953 A1**
(43) Date of publication of application: **11.10.2006**
(21) Application number: 05007379.0
(22) Date of filing: 05.04.2005
(51) Int. Cl.: A61K 8/29, A61Q 1/02

(54) **Composition comprising metal oxides**

(71) Applicant: THE PROCTER & GAMBLE COMPANY, Cincinnati, Ohio 45202 (US)
(72) Inventor: Elliott, Russell Phillip, Virginia Water Surrey, GU25 4EU (GB)
(74) Representative: Wilding, Richard Alan

(57) **Abstract**

A composition is provided comprising:
(a) first metal oxide particles having a first number weighted average primary particle size from 1nm to 50nm; and
(b) second metal oxide particles having a second number weighted average primary particle size which is greater than 50nm and less than or equal to 200nm;

wherein organo-functionalised silicone fibrils are bonded to and extend away from the surface of one or both of the first and second metal oxide particles.

## Description

### FIELD OF THE INVENTION

In a first aspect, the present application concerns compositions comprising metal oxide particles having at least two different average primary particle sizes, at least some of the particles being coated to prevent flocculation. In a second aspect, the present application concerns cosmetic compositions comprising compositions according to the first aspect of the invention.

### BACKGROUND OF THE INVENTION

It is known to incorporate metal oxide particles within compositions in order to provide a variety of different benefits, especially cosmetic benefits, such as UV-screening and colouring benefits as well as for obscuring skin defects (in the case of a skin foundation, for example). It is also known to incorporate metal oxide particles of differing particle sizes into cosmetic compositions in order to achieve a combination of benefits, such as both a sun-screening and a skin obscuring benefit - metal oxide sunscreen particles may have sizes below about 200nm, whereas metal oxide pigments have particle sizes above and typically well above 200nm. It is also known to incorporate metal oxide particles of differing particle sizes into cosmetic compositions in order to improve the degree of a single type of benefit. WO 90/11067 teaches to use a combination of two different titanium dioxide particle sizes to provide effective blocking of not only UVB, but also UVA radiation. The compositions according to WO 90/11067 would not be suitable for use as products (such as cosmetic skin foundations) designed to obscure the skin, however, because those compositions are specifically designed not to afford significant reflectance of visible light (they are designed to be transparent).

It is furthermore known to include fatty or oleophilic materials, including silicones, in cosmetic products to provide occlusive (moisture-retention) properties, improved feel properties, as solvents and for other reasons.

In order to marry the benefits of both metal oxides particles and oleophilic materials, it is a natural step to consider including both types of component in a single composition. This presents difficulties, however, because metal oxide particles generally do not readily disperse in a hydrophobic matrix. To overcome this difficulty, it is known to stabilise metal oxide particles within an oleophilic phase by adding emulsifying agents to a composition.

Emulsifiers in low dielectric constant media may use steric effects to provide stabilisation and prevent flocculation. To be more precise, the emulsifier coats the free surface of the particle with hydrophilic tails and extends oleophilic chain into the medium and the chain acts to prevent agglomeration by osmotic effects: as two particles approach one another, the chains overlap and cause a temporary increase in polymer concentration. This increase causes an osmotic stress that may force fluid between the particles, thereby causing them to separate.

The addition of free emulsifier to a composition may, however, not be sufficient to ensure a good particle dispersion throughout the life cycle of the product. If the concentration of emulsifier is too low then the osmotic stress will be correspondingly low and, as two particles approach one another, the emulsifier may bridge the particles actually promoting agglomeration. If, on the other hand, the concentration of emulsifier is too high, then the osmotic stress behaviour may be reversed leading to depletion flocculation. In this scenario, the concentration of free emulsifier may be so high that, as particles approach one another, free emulsifier may be forced out from between them. The concentration difference may create an osmotic stress that draws fluid out of the space between the particles thereby promoting agglomeration.

An ideal level of emulsifier exists for any system but small changes in that system may cause the amount of emulsifier to move away from the optimum, thereby leading to the above-described problems. Especially in the case of a product that is required to dry down in use, it is almost impossible to achieve an ideal level of emulsifier at all time points, because, during the drying process, the emulsifier concentration continually increases. In other words, the use of non-bonded coating on the surface of the particle means that for any system the emulsifier concentration must be sub-optimal at some stage in its life cycle.

To overcome the disadvantages of non-bonded emulsifier coatings, use of a coating which is bonded to the surface of a particle may be employed. Bonding may prevent the emulsifier bridging and, since the there is no free emulsifier in the solution, depletion flocculation may be avoided. In addition, since it is no longer necessary to prevent diffusion of emulsifier away from the surface, there is no requirement to control the hydrophilic/lipophilic balance (HLB) of the emulsifier. As a result, the molecules in a bonded coating may have longer tails than non-bonded emulsifiers, which, in turn, may increase the steric stabilisation effect.

It is known to formulate compositions comprising metal oxide particles which have been coated with bonded emulsifier to provide steric stabilisation. Such formulations are disclosed in the article entitled "Development of Novel Silicones for Powder Surface Treatment" by Masaneo Kamei in the Fragrance Journal, p.81-85, 2002-6. The cosmetic compositions disclosed in that article are difficult to apply to skin, however, which may result in the benefit achieved by the metal oxide particle benefit agent being less even than it should be.

### SUMMARY OF THE INVENTION

According to the invention, a composition is provided comprising:
(a) first metal oxide particles having a first number weighted average primary particle size from 1nm to 50nm; and
(b) second metal oxide particles having a second number weighted average primary particle size which is greater than 50nm and less than or equal to 200nm;
wherein organo-functionalised silicone fibrils are bonded to and extend away from the surface of one or both of the first and second metal oxide particles.

As used herein, the term "bond" includes, but is not limited to, chemical bonds, such as chemisorption and covalent bonds. The term "bonded" is to be interpreted accordingly.

According to a second aspect of the invention, cosmetic compositions are provided comprising compositions according to the first aspect of the invention.

### DETAILED DESCRIPTION OF THE INVENTION

All weights, measurements and concentrations herein are measured at 25°C on the composition in its entirety, unless otherwise specified.

As used herein in relation to metal oxide sunscreen particles, all weights of doping or coating materials are given as percentages of the weight of the underlying metal oxide particle which is thus doped or coated. This definition applies even when the doping or coating material is, itself, a metal oxide. Thus, if the particles weigh x grammes and the coating or doping material weighs y grammes, the percentage weight of the coating or doping material is y/x * 100.

As used herein in relation to the cosmetic composition, the percentage weight of the metal oxide sunscreen particles is the combined weight of the underlying metal oxide particle and any doping or coating divided by the weight of the entire cosmetic composition. Thus, if the particles weigh x grammes, the coating or doping material weighs y grammes and the entire cosmetic composition (including the coated or doped metal oxide particles) weighs z grammes, then the percentage weight of the metal oxide particle is (x + y)/z * 100.

Unless otherwise indicated, all percentages of compositions referred to herein are weight percentages of the total composition (i.e. the sum of all components present) and all ratios are weight ratios.

Unless otherwise indicated, all polymer molecular weights are number average molecular weights.

Reference herein to the percentage weight of cross-linked organopolysiloxane elastomer in a composition is a reference to the percentage weight of solid organopolysiloxane elastomer in that composition, not to the percentage weight of solid organopolysiloxane elastomer plus solvent in the composition. This is stated for the avoidance of doubt, since commercially available organopolysiloxane elastomers are often sold in combination with a solvent.

Unless otherwise indicated, the content of all literature sources referred to within this text are incorporated herein in full by reference.

Except where specific examples of actual measured values are presented, numerical values referred to herein should be considered to be qualified by the word "about".

Metal oxide primary particles in the nano- and micrometer size range have high surface areas per unit volume and are correspondingly reactive leading to their agglomeration to form secondary particles. This agglomeration is inevitable and, up to a point, may not be undesirable - minute, nanometer-size particles do not scatter light, making them less suitable as UV-A sunscreens. If the secondary particle size is unchecked, however, then the particles generated may not have the required properties and the composition may not provide an even benefit in use. In addition, particularly in the case of sunscreens, excessive agglomeration may alter or significantly reduce the benefit obtained: it is believed that the primary particle size drives the overall surface area of the secondary particles, with smaller primary particles generally giving rise to secondary particles of greater surface area. Secondary particle surface area, in turn, is believed to drive absorption of UV-B radiation - the larger the secondary particle surface area, the greater the degree of UV-B absorption. Secondary particle size, on the other hand, is considered to drive scattering of UV-A radiation, with larger particles scattering more. Without wishing to be bound by theory, it is believed that agglomeration of secondary particles may reduce both UV-B absorption and UV-A scattering, thereby significantly affecting the sunscreening benefit: agglomeration of secondary particles drives down overall secondary particle surface area, thereby reducing the degree of UV-B absorption; in addition, whilst UV-A reflection does increase with secondary particle size, the overall number of secondary particles drops with increasing agglomeration, thereby reducing UV-A reflection too.

The present invention requires the presence of metal oxide particles having at least two different number weighted average primary particle sizes. In this way, a range of UV scattering and absorbing effects may be achieved over a broad spectrum. It remains important, however, to address the question of agglomeration in hydrophobic media, which can give rise to the disadvantages described above.

In order to reduce or prevent agglomeration, the first and/or the second metal oxide particles are provided with a coating of organo-functionalised silicone fibrils, which fibrils are bonded to and extend away from the surface of the metal oxide particles into the carrier medium. As already discussed, it is believed that, as coated metal oxide particles approach one another, their fibrils may become enmeshed. The resulting high concentration of organo-functionalised siloxane polymer in the region where that enmeshing is occurring generates a high osmotic pressure causing carrier fluid to flow in between the adjacent particles and force them apart.

The fibrils may advantageously be attached by treating the metal oxide particles with an organo-functionalised silicone polymer comprising a reactive moiety selected from the group consisting of amino, imino, halogen, hydroxyl, and alkoxyl such that the organo-functionalised silicone polymer becomes adsorbed to the surface of the metal oxide polymer.

Advantageously, the organo-functionalised silicone polymer comprises from 5 to 100, preferably from 25 to 50 silicone repeating units. Polymers of this size project into and may flow freely in the carrier medium, thereby avoiding agglomeration further. As used herein, a "silicone repeating unit" or "silicone unit" means 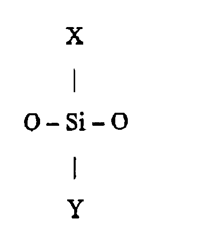 where each of X and Y is, independently, an alkyl group or any functional group.

Preferably, the organo-functionalised silicone polymer has a ratio (Mw/Mn) of weight-average molecular weight (Mw) to number-average molecular weight (Mn) from 1.0 to 1.3. Without wishing to be bound by theory, it is believed to be important that the surface coating be as even as possible to maximise the osmotic pressure and also to avoid bridging flocculation by comparatively longer polymer chains.

The organo-functionalised silicone polymer may be a linear organofunctionalised silicone polymer. In this case, it is preferred to locate the reactive moiety at one end of its molecular chain.

Alternatively, the organo-functionalised silicone polymer may be a branched chain organofunctionalised silicone polymer. In this case, the reactive moiety is preferably located on a side chain. Advantageously, the side chain on which the reactive moiety is found is located within five silicone repeating units, preferably within three silicone repeating units of one end of the silicone backbone.

To manufacture the coated metal oxide particles, organo-functionalised silicone polymer, as specified above, an organic solvent which dissolves said organo-functionalised silicone polymer, and metal oxide are mixed, then dried by heating. The organo-functionalised silicone polymer should be used in an amount from 0.1 wt% to 30 wt%, preferably from 1 wt% to 15 wt %, more preferably from 2 wt% to 8 wt% of the metal oxide particles to be treated, depending on its particle diameter and specific surface area.

An appropriate organic solvent should be selected in consideration of its flash point and ignition point, and the surface activity and heat stability of the metal oxide particles for surface treatment. Preferred examples of the organic solvent include ethers, ketones, halogenated hydrocarbons, aliphatic hydrocarbons, and alcohols and mixture thereof with other solvents such as water. The organic solvent should be used in an amount of 1-50 wt % to the metal oxide particles.

The mixing of the organo-functionalised silicone polymer, organic solvent, and metal oxide particles may be accomplished by putting them together into an ordinary mixer, or by spraying the organo-functionalised silicone polymer onto a mixture of the organic solvent and metal oxide particles. The heating of the mixture should be carried out in an adequate manner in consideration of the heat resistance of the metal oxide particles and the kind of organic solvent used.

Examples of suitable organo-functionalised silicone polymers include dimethylpolysiloxysilazane, α-monohydroxysiloxane, α,ω-dihydroxypolydimethylsiloxane, α-monoalkoxypolydimethylsiloxane, α,ω-dihdroxypotydimethylsiloxane, α-dialkoxypolydimethylsiloxane, α-trialkoxypolydimethylsiloxane, α,ω-hexa-alkoxypolydimethylsiloxane dimethylpolysiloxy chloride, dimethylpolysiloxy bromide, and dimethylpolysiloxy iodide. Preferred among those examples are .alpha.-monoalkoxypolydimethylsiloxane, .alpha.-dialkoxypolydimethylsiloxane, alpha.-trialkoxypolydimethylsiloxane, α-monohydroxymethylphenyl siloxane, α- trialkoxypolymethyl hexyl siloxane and methyl styryl/dimethyl polysiloxy bromide. They are adsorbed to the pigment very easily, and upon adsorption they impart a smooth feel to the treated pigment. The reactive group in the organo-functionalised silicone may be joined to the silicon atom directly or indirectly thorough a substituent group.

Commercially available organo-functionalised silicone polymers which may be employed to coat the metal oxide particles include the following materials: X-24-9826, X-24-9171 and X-24-9174 manufactured by the Shin Etsu Co. Ltd; TSL 8185 and TSL 8186 manufactured by Toshiba Silicone Co. Ltd.; S106645.0 manufactured by Chisso Corporation; KBM-3103 manufactured by Shin-Etsu Chemical Co. Ltd.; A-137 manufactured by Nippon Unicar Co. Ltd.

In addition to providing the metal oxide particles with fibrils, they may also be provided with a hydrophobic coating to improve the particles' dispersion in hydrophobic carrier medium. The hydrophobic coating may be applied as a pre-treatment, prior to provision of the fibrils, or as a post-treatment, after provision of the fibrils. Advantageously, the metal oxide particles comprise from 2 to 25%, preferably from 5% to 15%, more preferably from 7% to 12% hydrophobic coating by weight of the metal oxide particles.

Advantageously, the hydrophobic coating may be made by applying a mixture of one or more of the following materials and isopropyl alcohol onto the metal oxide powder and drying at 150°C for 3 hours: reactive organo-polysiloxane, polyolefin (including polyethylene and polypropylene), hydrogenated lecithin and salts thereof, N-acylamino acid and salts thereof and dextrin fatty acid esters. Preferably, the reactive organo-polysiloxane comprises organo hydrogen polysiloxane, triorgano siloxy silicic acid and organopolysiloxane modified at both terminal ends with trialkoxy groups. Commercially available materials falling into the category of reactive organo-polysiloxanes include KF-99, KF-9901, KF-7312F, KF-7312-J, KF-7312K, KF-9001, KF-9002, X-21-5249 and X-21-5250 manufactured by the Shin-Etsu Chemical Company Ltd; SH-1107, DC593, BY-11-015,BY-11-018 and BY-11-022 manufactured by Dow Coming Toray Silicone Co. Ltd.; TSF484, TSF483 and TSF4600 manufactured by Toshiba Silicone Co. Ltd.; FZ3704 and AZ6200 manufactured by Nippon Unicar Co. Ltd.

The hydrophobic coating is not limited to those described in the preceding paragraph and alternative hydrophobic coatings known to the skilled person may be employed instead. Such coatings may include trialkoyl isopropyl titanate, preferably triisostearoyl isopropyl titanate and perfluoro coatings, preferably polyperfluoroethoxymethoxy PEG-2 phosphate.

Some coatings may both provide hydrophobic properties and exhibit fibrils to provide steric stabilisation to avoid flocculation. Commercially available coatings falling into this category include KF9908 (Triethoxysilylethyl Polydimethylsiloxyethyl Dimethicone), KF9909 (Triethoxysilylethyl Polydimethylsiloxyethyl Hexyl Dimethicone) and KP575 (Acrylate/Tridecyl Acrylate/Triethoxysilylproplyl Methacrylate/Dimethicone Methacrylate Copolymer) from the Shin Etsu Co Ltd.

An essential feature of the compositions according to the invention is the presence of first metal oxide particles having a first number weighted average primary particle size from 1nm to 50nm and second metal oxide particles having a second number weighted average primary particle size which is greater than 50nm and less than or equal to 200nm. Optionally, one or more additional metal oxide particles may also be present, the or each of the additional metal oxide particles having a number weighted average primary particle size that differs from that of the first, second and any other metal oxide particles present.

The first and second metal oxide particles according to the invention may comprise any suitable metal oxides and each of the first and second metal oxides may be oxides of a single metal or mixtures of two or more metals (provided that the number weighted average primary particle size conditions are adhered to). Preferably, the first and second metal oxide particles are selected from the group consisting of titanium oxide, zinc oxide, zirconium oxide, yellow iron oxide, black iron oxide, red iron oxide, chromium oxide, chromium hydroxide, zirconium oxide, cerium oxide and mixtures thereof. More preferably, the first and second metal oxide particles are selected from titanium dioxide particles, zinc oxide particles and mixtures thereof. More preferably still, the first and second metal oxide particles comprise titanium dioxide particles.

As discussed above, the primary particle size is important in determining the surface area of the secondary particles and, according to the invention, the first metal oxide particles have a first number weighted average primary particle size from 1nm to 50nm and the second metal oxide particles have a second number weighted average primary particle size which is greater than 50nm and less than or equal to 200nm. Advantageously, the first number weighted average primary particle size is from 5 to 40nm, preferably from 8 to 30nm. Advantageously, the second number weighted average primary particle sizes is greater than 50nm and less than or equal to 150nm, preferably it is from greater than 50nm and less than or equal to 120nm and more preferably it is from 55nm to 100nm. By having both the defined smaller and larger particles present, three objectives may be achieved simultaneously - a UVB sunscreening effect, a UVA sunscreening effect and improved skin coverage due to increased reflection of visible light by the larger particles.

In addition, the first metal oxide particles advantageously comprise from 0.1 to 10%, preferably from 0.5 to 8%, more preferably from 1 to 5% of the composition and the second metal oxide particles advantageously comprise from 0.1 to 10%, preferably from 0.5 to 8%, more preferably from 2 to 5% of the composition.

Advantageously, compositions according to the present invention comprise a higher percentage weight of the second metal oxide particles than of first metal oxide particles such that the weight ratio:
weight of first metal oxide particles
weight of second metal oxide particles
is less than 1.0 and is in the range 0.99 to 0.1. This may be especially advantageous in the case of cosmetic compositions which are intended to mask the underlying skin to some degree, such as cosmetic skin foundations: by having more of the larger particles present, three objectives may be achieved simultaneously - a UVB sunscreening effect, a UVA sunscreening effect and improved skin coverage due to increased reflection of visible light by the higher proportion of larger particles.

Commercially available materials which may be employed as first metal oxide particles include M262 from Kemira Oy. and TTO S-3, TTO S-4 , TTO V-3 from Ishihara Corp. Commercially available materials which may be employed as second metal oxide particles include KQ-1, MPT-140 and MPT141 from Ishihara Corp. In a preferred embodiment, SAS-TTO S-3/D5 from Miyoshi Kasei (which comprises fibril-coated TTO S-3 particles), which have an average primary particle of about 15nm, and SAS-KQ-1 (which comprises fibril-coated KQ-1 particles), with a primary particle size of about 60nm, may be combined as first and second metal oxide particles respectively.

As used herein, the term "primary particle size" means metal oxide crystal size, as determined by x-ray diffraction. It is based on measuring the broadening of the strongest rutile line.

Advantageously, the compositions of the present invention are cosmetic compositions and highly advantageously, they are cosmetic skin foundations.

Such cosmetic compositions may comprise from 0.1 wt% to 50 wt% combined metal oxide particles consisting of first metal oxide particles, second metal oxide particles and, optionally, from 0 to 30% of one or more additional metal oxide particles, the or each of the additional metal oxide particles having a number weighted average primary particle size that differs from that of the first, second and any other metal oxide particles present. If, for example, metal oxide pigments are present, then the cosmetic composition preferably comprises from 0.05 wt% to 30 wt%, preferably from 1 wt% to 20 wt% metal oxide pigments.

Metal oxide pigments that may be employed include Anatase pigments, such as X200 from Kemira Oy (Finland) and Rutile pigments, such as CR50 or PF 671 from ISK Japan. X200 is available as a 65% methicone treated dispersion from Kobo Products Inc as FA65UMLO.

In a preferred embodiment, SAS-TTO S-3/D5, SAS-KQ-1 and FA65UMLO may be combined as first, second and third metal oxide particles, respectively.

Minute metal oxide particles have a highly reactive surface that can cause unwarranted chemical or photochemical reactions. To counter this effect, it is known to dope these surfaces with one or more other materials such as silica, or metal oxides, such as alumina, to reduce the reactivity of the surface. This surface treatment may typically represent from 15 to 30% by weight of the metal oxide particle. Advantageously metal oxide particles comprised within cosmetic compositions according to the invention may be so-doped.

Lastly, for the case in which the metal oxide particles are titanium dioxide, then those titanium dioxide particles are advantageously manufactured by the titanium tetrachloride process - the "Chloride Process". Especially preferred are those materials which, in addition, have been calcined. While not wishing to be bound by theory it is believed that titanium dioxide particles manufactured by the Chloride Process, especially if it has been calcined, give rise to reduced porosity and require a lower amount of coatings to bestow hydrophobicity and/or porevent agglomeration.

Advantageously, the cosmetic compositions according to the invention comprise a hydrophobic phase. The hydrophobic phase may comprise cross-linkedorganopolysiloxane elastomers, oils, gums, waxes and other hydrophobic materials. Preferably, the hydrophobic phase comprises cross-linked organopolysiloxane elastomer.

Cross-linked organopolysiloxane elastomer may be present in an amount which is greater than 1.7% up to 15%, preferably from 2% to 10%, more preferably from 2.2 to 5% by weight of the cosmetic composition. As stated above, these are the amounts of solid organopolysiloxane elastomer in the composition. Surprisingly, the present inventors have established that above 1.7% elastomer, and especially in the preferred ranges, the evenness of the benefit provided by the metal oxide particles improves.

The compositions of the present invention may comprise emulsifying cross-linked organopolysiloxane elastomer, non-emulsifying cross-linked organopolysiloxane elastomer or mixtures thereof. If present, then the emulsifying cross-linked organopolysiloxane elastomer is present in an amount from 0.01 to 15%, preferably from 0.01 to 1% by weight of the composition. In addition and if present, the non-emulsifying cross-linked organopolysiloxane elastomer is advantageously present in an amount from 0.01 to 15%, preferably from 2-5% by weight of the cosmetic composition.

As used herein, the term "non-emulsifying" when employed in relation to cross-linked organopolysiloxane elastomer includes cross-linked organopolysiloxane elastomer which comprise no polyoxyalkylene or polyglyceryl units.

As used herein, the term "emulsifying" when employed in relation to cross-linked organopolysiloxane elastomer includes cross-linked organopolysiloxane elastomer which comprise at least one polyoxyalkylene (e.g., polyoxyethylene or polyoxypropylene) or polygyceryl unit.

No specific restriction exists as to the type of curable organopolysiloxane composition that can serve as starting material for the cross-linked organopolysiloxane elastomer. Examples in this respect are addition reaction-curing organopolysiloxane compositions which cure under platinum metal catalysis by the addition reaction between SiH-containing diorganopolysiloxane and organopolysiloxane having silicon-bonded vinyl groups; condensation-curing organopolysiloxane compositions which cure in the presence of an organotin compound by a dehydrogenation reaction between hydroxyl-terminated diorganopolysiloxane and SiH-containing diorganopolysiloxane; condensation-curing organopolysiloxane compositions which cure in the presence of an organotin compound or a titanate ester, by a condensation reaction between an hydroxyl-terminated diorganopolysiloxane and a hydrolyzable organosilane (this condensation reaction is exemplified by dehydration, alcohol-liberating, oxime-liberating, amine-liberating, amide-liberating, carboxyl-liberating, and ketone-liberating reactions); peroxide-curing organopolysiloxane compositions which thermally cure in the presence of an organoperoxide catalyst; and organopolysiloxane compositions which are cured by high-energy radiation, such as by gamma-rays, ultraviolet radiation, or electron beams.

Preferred non-emulsifying organopolysiloxane compositions are dimethicone/vinyl dimethicone crosspolymers. Such dimethicone/vinyl dimethicone crosspolymers are supplied by a variety of suppliers including Dow Coming (DC 9040, DC 9041), General Electric (SFE 839 and Velvesil materials), Shin Etsu (KSG-15, 16, 18 [dimethicone/phenyl vinyl dimethicone crosspolymer]), Grant Industries (Gransil(TM) line of materials), lauryl dimethicone/vinyl dimethicone crosspolymers supplied by Shin Etsu (e.g. KSG-41, KSG-42, KSG-43, and KSG-44).

Particularly useful emulsifying elastomers are polyoxyalkylene-modified elastomers formed from divinyl compounds, particularly siloxane polymers with at least two free vinyl groups, reacting with Si-H linkages on a polysiloxane backbone. Preferably, the elastomers are dimethyl polysiloxanes cross-linked by Si-H sites on a molecularly spherical MQ resin. Examples of commercially available emulsifying cross-linked organopolysiloxane elastomers include KSG-21 (comprising 27% solid organopolysiloxane elastomer), KSG-210 (comprising 24% solid organopolysiloxane elastomer), KSG-240 and KSG-310, KSG-320 from the Shin-Etsu Chemical Company Ltd. Commercially available examples of emulsifying cross-linked organopolysiloxane elastomers comprising polyglyceryl units are KSG 710 and KSG-800 from the Shin-Etsu Chemical Company Ltd.

Advantageously, cosmetic compositions according to the invention may comprise oil. Oil may be present in an amount from 7% to 80% by weight of the cosmetic composition. The oil may be selected from the group consisting of volatile oils, non-volatile oils and mixtures thereof.

As used herein, the term "non-volatile" when employed in relation to an oil includes oils that fulfil at least one of the following definitions: (a) the oil exhibits a vapour pressure of no more than about 0.2 mm Hg at 25°C and one atmosphere pressure; (b) the oil has a boiling point at one atmosphere of at least about 300°C.

As used herein, the term "volatile" when employed in relation to oils includes materials that are not "non-volatile" as previously defined herein.

Any non-volatile oil adhering to the above definition may be included in cosmetic compositions according to the invention. Such non-volatile oils may include silicone oils, both functionalised and non-functionalised, hydrocarbon oils and mixtures thereof. Non-volatile oil may be present in an amount from 0 to 20%, preferably from 1 to 10% by weight of the cosmetic composition.

Volatile oils which may be included in cosmetic compositions according to the invention may include silicone oils, both functionalised and non-functionalised, hydrocarbon oils and mixtures thereof. Volatile oil useful in the present invention may exhibit one or more of the following characteristics - it may be saturated or unsaturated, have a straight or branched chain or a cyclic structure.

Examples of volatile hydrocarbons which may be incorporated into cosmetic compositions according to the invention include polydecanes such as isododecane and isodecane (e.g., Permethyl-99A which is available from Presperse Inc.) and the C₇-C₁₅ isoparaffins (such as the Isopar Series available from Exxon Chemicals).

Examples of volatile silicone oils which may be incorporated into cosmetic compositions according to the invention include cyclic volatile silicones corresponding to the formula: 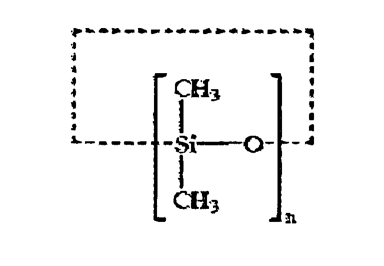 wherein n is from about 3 to about 7 and linear volatile silicones corresponding to the formula:

(CH₃)₃Si-O-[Si(CH₃)₂-O]ₘ-Si(CH₃)₃

wherein m is from about 1 to about 20 preferably from 3 to 12.

Preferably, the cyclic volatile silicones are selected from cyclopentasiloxane, cyclohexasiloxane and mixtures thereof.

Linear volatile silicones generally have a viscosity of less than about 5 centistokes at 25°C; cyclic silicones generally have viscosities of less than about 10 centistokes at 25°C.

Examples of commercially available volatile silicone oils include the following cyclomethicones: Dow Coming 200, Dow Coming 244, Dow Coming 245, Dow Coming 344, and Dow Coming 345 (commercially available from Dow Coming Corp.); SF-1204 and SF-1202 Silicone Fluids (commercially available from G. E. Silicones), GE 7207 and 7158 (commercially available from General Electric Co.); and SWS-03314 (commercially available from SWS Silicones Corp.). Other examples of commercially available methyl silsesquioxanes available as TMF 1.5 fluid from Shin-Etsu Chemical Co; SILCARE SILICONES, for example phenyl substituted silsesquioxanes available as Silcare 15M60, n-Octyl substituted silsesquioxanes available as Silcare 31M60 and 31M70, hexyl methicone, caprylyl methicone and lauryl methicone available as Silcare 41M10, 41M15 and 41M20 respectively from Clariant.

Volatile oil may be present in an amount from 7 to 70%, preferably from 10% to 50%, more preferably 20% to 40% by weight of the cosmetic composition.

In one advantageous embodiment, it is preferred that the volatile oil comprise a mixture of volatile cyclic silicone and volatile linear dimethicone of viscosity from 2 to 50 x 10⁻⁶ m²/s (2-50cst), more preferably from 3 to 50 x 10⁻⁶m²/s (3-5cst), more preferably still from 3 to 50 x 10⁻⁶m²/s (4cst). Without wishing to be bound by theory it is believed that, during dry-down the linear dimethicone may remain on the skin longer to keep the metal oxide particles wetted, thereby reducing agglomeration. Agglomeration is responsible for colour drift, in the case of pigments, and reduced SPF efficacy, in the case of sunscreens.

Advantageously, the ratio of volatile cyclic silicone to volatile linear dimethicone is from 1:1 to 25:1, preferably from 5:1 to 10:1.

Preferred examples of linear dimethicones useful include DC200 5cst, DC1630 and DC 5-2117, More preferably, the linear dimethicone comprises DC 5-2117.

Cosmetic compositions according to the invention may be formulated as anhydrous products or as emulsions. If the cosmetic compositions are formulated as emulsions, those emulsions may be water-in-oil (water-in-silicone) emulsions or oil-in-water (silicone-in-water) emulsions, but are preferably water-in silicone emulsions.

Advantageously, the cosmetic compositions according to the invention are formulated as water-in-silicone emulsions that contain from 0.1 to 70%, preferably from 1 to 50%, more preferably from 5 to 40% water.

Cosmetic compositions according to the invention, whether or not they are in the form of an emulsion, may comprise emulsifier. The emulsifier may be selected from the group consisting of nonionic, anionic, cationic, zwitterionic and amphoteric emulsifiers and mixtures thereof. Suitable emulsifiers are disclosed in McCutcheon's Detergents and Emulsifiers, North American Edition, pages 317-324.

In the event that the cosmetic composition according to the invention is a water-in-silicone emulsion, then preferred emulsifiers are selected from the group consisting of polyoxyalkylene copolymers (also known as silicone polyethers), polyglyceryl copolymers and mixtures thereof. Polyoxyalkylene copolymers are described in detail in US 4,268,499. More preferred polyethers include PEG/PPG-18/18 Dimethicone available as blend with cyclopentasiloxane as DC5225C or DC5185; PEG 9 Dimethicone, available as KF6017 or KF6028 from Shin-Etsu. A preferred polyglyceryl emulsifier is available as KF6100 and KF6104 from Shin-Etsu Inc.

In one embodiment, it is preferred that cosmetic compositions according to the invention comprise only polyglyceryl copolymer emulsifiers and no polyoxyalkylene emulsifiers. This is because polyoxyalkylene emulsifiers may break down to release ethylene glycol and aldehydes which may give rise to increased sensitivity on the skin of some consumers.

The total concentration of the emulsifier may be from 0.01% to about 15%, more preferably from about 0.1% to about 10% of the formulation, even more preferably from 1.0% to about 5% and more preferably still from about 1.0% to about 3%, by weight of the composition.

Cosmetic compositions according to the present invention may optionally contain spherical particles having an average particle diameter from 1 to 50 µm, preferably from 5 to 20 µm. As used herein in relation to the spherical particles, the particle diameter shall be understood to be that of primary particles.

Preferred spherical particles include, but are not limited, to polymeric particles chosen from the methylsilsesquioxane resin microspheres such as for example those sold by GE silicone under the name Tospearl 145A or Tospearl 2000; microspheres of polymethylmethacrylates such as those sold by Seppic under the name Micropearl M 100; the spherical particles of crosslinked polydimethylsiloxanes, especially such as those sold by Dow Corning Toray Silicone under the name Trefil E 506C or Trefil E 505C, sphericle particles of polyamide and more specifically Nylon 12, especially such as those sold by Atochem under the name Orgasol 2002D Nat Cos, polystyerene microspheres such as for example those sold by Dyno Particles under the name Dynospheres, ethylene acrylate copolymer sold by Kobo under the name FloBead EA209 and mixtures thereof. Ronasphere LDP from Kobo Inc., polyurethane particles BPD500 sold by Kobo Inc. and Ganzpearl GME-0830, methylmethacrylate crosspolymer sold by Ganz Inc. may also be employed.

If present, the spherical particles may be included in the cosmetic compositions according to the invention at a concentration of from about 0.01% to about 40%, more preferably from about 1% to about 10%, more preferably still from about 1% to about 5%.

Cosmetic compositions according to the present invention may further comprise a skin-conditioning agent. These agents may be selected from humectants, exfoliants or emollients and may be present from about 0.01% to 30%, preferably from about 1% to about 20%, more preferably from about 1% to 10% by weight of the cosmetic composition.

Humectants which may be included in cosmetic compositions according to the invention include polyhydric alcohols such as glycerine, propylene glycol, dipropylene glycol, polypropylene glycol, polyethylene glycol, sorbitol, hydroxypropyl sorbitol, hexylene glycol, 1,3-butylene glycol, 1,2,6-hexanetriol, ethoxylated glycerin, propoxylated glycerine and mixtures thereof. Most preferably the humectant comprises glycerine.

In addition, hydrophilic gelling agents such as those selected from the group consisting of the acrylic acid/ethyl acrylate copolymers, carboxyvinyl polymers (such as those sold by the B.F. Goodrich Company under the Carbopol trademark, polyacrylamides (such as those available from Seppic as Seppigel 305) and mixtures thereof may be included in the cosmetic compositions according to the invention.

Cosmetic compositions according to the present invention may additionally comprise an organic sunscreen. The exact amount of the sunscreen active will vary depending upon the desired Sun Protection Factor, i.e., the "SPF" of the composition as well as the desired level of UVA protection. The compositions of the present invention preferably comprise an SPF of at least 10, preferably at least 15. SPF is a commonly used measure of photoprotection of a sunscreen against erythema. The SPF is defined as a ratio of the ultraviolet energy required to produce minimal erythema on protected skin to that required to products the same minimal erythema on unprotected skin in the same individual (see Federal Register, Vol. 64, No 98, pp. 27666 - 27693, 21 May, 1999).

Cosmetic compositions according to the present invention may comprise from about 2% to about 20%, preferably from about 4% to about 14%, by weight, of organic sunscreen. Suitable sunscreens include, but are not limited to, those found in the CTFA International Cosmetic Ingredient Dictionary and Handbook, 7th edition, volume 2 pp. 1672, edited by Wenninger and McEwen (The Cosmetic, Toiletry, and Fragrance Association, Inc., Washington, D. C., 1997). It is particularly preferred to include water soluble or water dispersible organic sunscreens in the present composition. Non-limiting examples of water soluble organic sunscreens include PBSA (2-Phenylbenzimidazole-5-sulfonic acid), Benzophenone-4 (2-Benzoyl-5-Methoxy-1-Phenol-4-Sulphonic Acid) and PABA (Para Amino Benzoic Acid). A non-limiting example of a water dispersible organic sunscreen is methylene bis-benzotriazolyl tetramethylbutylphenol (Available in a 50% dispersion as Tinosorb M from Ciba).

A variety of additional optional ingredients may be incorporated into the compositions of the present invention. Non-limiting examples of these additional ingredients include additional skin care actives such as peptides (e.g., Matrixyl [pentapetide derivative]), farnesol, bisabolol, phytantriol, urea, guanidine (e.g., amino guanidine); vitamins and derivatives thereof such ascorbic acid, vitamin A (e.g., retinoid derivatives such as retinyl palmitate or retinyl proprionate), vitamin E (e.g., tocopherol acetate), vitamin B₃ (e.g., niacinamide) and vitamin B₅ (e.g., panthenol) and the like and mixtures thereof; anti-acne medicaments (resorcinol, salicylic acid, and the like; antioxidants (e.g., phytosterols, lipoic acid); flavonoids (e.g., isoflavones, phytoestrogens); skin soothing and healing agents such as aloe vera extract, allantoin and the like; chelators and sequestrants; and agents suitable for aesthetic purposes such as essential oils, fragrances, skin sensates, opacifiers, aromatic compounds (e.g., clove oil, menthol, camphor, eucalyptus oil, and eugenol).

### Examples

The following examples further describe and demonstrate the preferred embodiments within the scope of the present invention. The examples are given solely for the purpose of illustration, and are not to be construed as limitations of the present invention since many variations thereof are possible without departing from its scope.

A liquid foundation of the present invention is prepared as follows: in a suitable vessel, water, glycerine, disodium EDTA and benzyl alcohol are added and mixed using conventional technology until a clear water phase is achieved. When the water phase is clear, the methylparabens are added and mixed again until clear. The resultant phase is mixed with a Silverson SL2T or similar equipment on high speed (8,000 rpm, standard head). In a separate vessel, the KSG21, DC245, Pigment dispersion, other oils and the parabens are added and the mixture is milled using a Silverson SL2T on a high speed setting until a homogeneous mixture is created.

Following this step, the water phase and the silicone phase are combined and milled using the Silverson SL2T on a high speed setting until the water is fully incorporated and an emulsion is formed. The elastomer is then added and the mixture is mixed again using the Silverson on a high speed setting to generate the final product.

| **Example #** | **1** | **2** | **3** | **4** | **5** | **6** | **7** | **8** |
|---|---|---|---|---|---|---|---|---|
| **Ingredient** | | | | | | | | |
| **DC9040 cross linked elastomer gel**^{**1**} | 25.00 | 20.00 | | | 15.00 | 50.00 | 30.00 | |
| **KSG15 cross linked elastomer gel**^{**2**} | | | 20.00 | 50.00 | | | | 17.00 |
| **Dimethicone copolyol cross-polymer (KSG21)**^{**3**} | | 5.00 | 10.00 | | | | | |
| **Cyclomethicone (DC245)** | 10.00 | 5.00 | 3.00 | 28.00 | | | | |
| **PEG/PPG18/18 Dimethicone & Cyclomethicone (DC5185)** | 1.8 | 2.0 | | | | | | |
| **Octyl Methoxy cinnamate** | 4.00 | | | | | | | |
| **2-Phenylbenzimidazole-5-sulfonic acid** | | | | 3.00 | | | | |
| **Triethanolamine** | | | | 3.00 | | | | |
| **Diethylhexyl carbonate (Tegosoft DEC)** | 4.00 | | | | 2.00 | 3.00 | | |
| **4cst Dimethicone (DC5-2117)** | | 4.00 | | 1.90 | | | | 4.00 |
| **Fibril coated sunscreen grade Titanium dioxide 50% dispersion SAS/TT0-S-3/D5** | 6.00 | | 4.00 | 6.00 | | 8.00 | 6.00 | |
| **Fibril coated sunscreen grade Titanium dioxide 50% dispersion SAS/TTO-V-3/D5** | | 5.00 | | | 3.00 | | | 5.00 |
| **Fibril coated pigmentary grade Titanium dioxide 60% Dispersion SAS KQ-1/** | 5.00 | 6.00 | | 8.00 | | 20.00 | | |
| **Fibril coated pigmentary grade Titanium dioxide 60% Dispersion SAS- MPT-140/** | | | 6.00 | | 8.00 | | | |
| **Fibril coated pigmentary grade Titanium dioxide 60% Dispersion SAS- MPT-141** | | | | | | | 8.00 | 10.00 |
| **Fibril coated pigment 50% dispersion SAS-CR-50/D5** | | | | 2.00 | 2.00 | | | |
| **3.00 Pigmentary Titanium Dioxide 65% Dispersion FA65UMLO** | 9.00 | 7.00 | 10.00 | | | | 2.0 | |
| **Iron oxides** | 1.50 | 1.50 | 1.50 | 2.00 | 2.00 | 3.00 | 1.50 | 2.00 |
| **Propylparabens** | 0.1 | 0.1 | 0.1 | 0.1 | 0.1 | 0.1 | 0.1 | 0.1 |
| **Ethylparabens** | 0.1 | 0.1 | 0.1 | 0.1 | 0.1 | 0.1 | 0.1 | 0.1 |
| **Methylparabens** | 0.1 | 0.1 | 0.1 | 0.1 | 0.1 | 0.1 | 0.1 | 0.1 |
| **Disodium EDTA** | 0.1 | 0.1 | 0.1 | 0.1 | 0.1 | 0.1 | 0.1 | 0.1 |
| **Benzyl alcohol** | 0.5 | 0.5 | 0.5 | 0.5 | 0.5 | 0.5 | 0.5 | 0.5 |
| **Sodium chloride** | 2.00 | 2.00 | 2.00 | 2.00 | 2.00 | 2.00 | 2.00 | 2.00 |
| **Glycerin** | 10.00 | 12.00 | 7.00 | 8.00 | 4.00 | 3.00 | 2.00 | 7.50 |
| **Niacinamide** | 2.00 | 5.00 | 5.00 | 3.00 | 5.00 | 1.00 | 0.00 | 0.00 |
| **Water** | qs | qs | qs | qs | qs | qs | qs | qs |

| | | | | | | | | |
|---|---|---|---|---|---|---|---|---|
| ¹DC9040 comprises 11% solid organopolysiloxane elastomer in cyclopentasiloxane. ²KSG15 comprises 9% solid organopolysiloxane elastomer in cyclopentasiloxane. ³KSG21 comprises 27% solid organopolysiloxane elastomer in cyclopentasiloxane. | | | | | | | | |

## Claims

1. A composition comprising:
(a) first metal oxide particles having a first number weighted average primary particle size from 1nm to 30nm; and
(b) second metal oxide particles having a second number weighted average primary particle size which is greater than 50nm and less than or equal to 200nm;
wherein organo-functionalised silicone fibrils are bonded to and extend away from the surface of one or both of the first and second metal oxide particles.

2. The composition of claim 1, wherein organo-functionalised silicone fibrils are bonded to and extend away from the surface of both the first and second metal oxide particles.

3. The composition of claim 1 or 2, wherein the fibrils are attached by treating the metal oxide particles with an organo-functionalised silicone polymer comprising a reactive moiety selected from the group consisting of amino, imino, halogen, hydroxyl, and alkoxyl.

4. The composition of claim 3, wherein the organo-functionalised silicone polymer comprises from 5 to 100, preferably from 25 to 50 silicone units.

5. The composition of claim 3 or 4, wherein the organo-functionalised silicone polymer has a ratio (Mw/Mn) of weight-average molecular weight (Mw) to number-average molecular weight (Mn) from 1.0 to 1.3.

6. The composition of any one of claims 3 to 5, wherein the organo-functionalised silicone is a linear organofunctionalised silicone.

7. The composition of claim 6, wherein the reactive moiety is located at one end of its molecular chain.

8. The composition of any one of claims 3 to 5, wherein the organo-functionalised silicone is a branched chain organofunctionalised silicone.

9. The composition of claim 8, wherein the reactive moiety is located on a side chain.

10. The composition of claim 9, wherein the side chain on which the reactive moiety is found is located within five silicone repeating units, preferably within three silicone repeating units of one end of the silicone backbone.

11. The composition of any one of the preceding claims, wherein the first metal oxide particles and the second metal oxide particles are selected from the group consisting of titanium dioxide, zinc oxide, cerium oxide, zirconium oxide, iron oxide particles and mixtures thereof

12. The composition of any one of the preceding claims, wherein the first number weighted average primary particle size is from 5 to 40nm, preferably from 8 to 30nm.

13. The composition of any one of the preceding claims, wherein the second number weighted average primary particle size is greater than 50nm and less than or equal to 150nm, preferably greater than 50nm and less than or equal to 120nm, more preferably from 55 to 100nm.

14. The composition of any one of the preceding claims, comprising from 0.1 to 10%, preferably from 0.5 to 8%, more preferably from 1 to 5% by weight of the composition of first metal oxide particles.

15. The composition of any one of the preceding claims, comprising from 0.1 to 10%, preferably from 0.5 to 8%, more preferably from 2 to 5% by weight of the composition of second metal oxide particles.

16. The composition of any one of the preceding claims, wherein the weight ratio:
weight of first metal oxide particles
weight of second metal oxide particles
is less than 1.0 and is preferably in the range 0.99 to 0.1.

17. The composition of any one of claims 1 to 16 which is a cosmetic composition, preferably a cosmetic skin foundation.

18. The cosmetic composition of claim 17, comprising a lipophilic phase.

19. The cosmetic composition of claim 17 or 18, comprising from above 1.7% to 15%, preferably from 2% to 10%, more preferably from 2.2% to 5% by weight of the cosmetic composition of cross-linked organopolysiloxane elastomer.

20. The cosmetic composition of any one of claims 17 to 19, comprising from 0.01 to 15%, preferably from 2% to 5% by weight of the cosmetic composition of non-emulsifying cross-linked organopolysiloxane elastomer.

21. The cosmetic composition of any one of claims 17 to 20, additionally comprising organic sunscreen, preferably a water soluble or water dispersible organic sunscreen.

22. Use of the composition of any one of claims 1 to 21 as a sunscreen.
